# EUROPÄISCHE PATENTANMELDUNG

(11) **EP 0 657 406 A1**
(43) Veröffentlichungstag der Anmeldung: **14.06.1995**
(21) Anmeldenummer: 94118397.2
(22) Anmeldetag: 23.11.1994
(51) Int. Cl.: C07C 7/11

(54) **Verfahren zur Trennung von Propylen-Propan-Gemischen**

(30) Priorität: 01.12.1993 DE 4340881
(71) Anmelder: HOECHST AKTIENGESELLSCHAFT, D-65926 Frankfurt am Main (DE)
(72) Erfinder: Bahrmann, Dr. Helmut, D-46499 Hamminkeln (DE); Höfs, Wolfgang, D-46147 Oberhausen (DE); Schmutzer, Hartwig, D-46149 Oberhausen (DE); Turon, Carlos, Colonia del Valle CP 03100 Mexico DF (MX)

(57) **Zusammenfassung**

Zur Trennung von Propylen-Propan-Gemischen wird der ungesättigte Kohlenwasserstoff mit Wasser absorbiert. Die Absorption kann durch Zusatz von Phasentransferreagentien zum Wasser unterstützt werden.

## Beschreibung

Die Erfindung betrifft ein Verfahren zur Trennung von Propylen-Propan-Gemischen durch selektive Absorption. Als Absorptionsmittel dient Wasser, dem Phasentransferreagenzien (Lösungsvermittler) zugesetzt sein können.

Die Trennung von Propylen-Propan-Gemischen ist ein Aufgabe, die sich in der Raffinerietechnik ebenso wie in der chemischen Industrie häufig stellt. Ihr Ziel ist entweder die Gewinnung der reinen Kohlenwasserstoffe oder aber die Rückgewinnung einer der beiden Komponenten, insbesondere des Propylens, z.B. aus Abgasen.

Je nach der geforderten Reinheit, in der die Bestandteile aus ihren Mischungen erhalten werden sollen, wendet man unterschiedliche Verfahren an. So kondensiert man nach dem Verfahren der DE-A-3 412 336 partiell das Abgas der Hydroformylierung von Propylen, es enthält üblicherweise 25 bis 40 Vol.-% Propylen, 6 bis 20 Vol.-% Propan und daneben Wasserstoff und Kohlenmonoxid, rektifiziert das Kondensat und führt die Propylenfraktion in die Reaktionsstufe zurück.

Die Extraktivdestillation von Propylen-Propan-Gemischen ist in Advan. Chem. Ser. 1972, Seiten 16 bis 34 als Alternative zur einfachen Destillation beschrieben. Extraktionsmittel sind u.a. Aceton, Furfural und Nitrile wie Acetonitril, Propionitril, Butyronitril und Benzonitril. Kennzeichnend für die Extraktivdestillation ist die Beeinflussung des Flüssig-Dampf-Gleichgewichtes des Propylen-Propan-Gemisches durch ein geeignetes Lösungsmittel ähnlich der Trägerdampfdestillation. Für die technische Durchführung ist eine Rektifikationskolonne mit Verdampfung im Sumpf und Rücklauf am Kopf typisch. Bei dem beschriebenen Verfahren wird Propan über Kopf abgezogen. Propylen ist im Lösungsmittel gelost und wird entweder in einer zweiten Rektifikationsstufe oder, falls eine Mischungslücke vorhanden ist, durch Phasentrennung gewonnen. Die Autoren kommen zum Schluß, daß die Extraktivdestillation gegenüber der üblichen Destillation nur in Sonderfällen Vorteile hat.

Ein weiteres Verfahren verwendet zur Abtrennung von Propylen aus Propylen-Propan-Mischungen beliebiger Zusammensetzung Molekularsiebe (DD-A-150 885). Es beruht darauf, daß Propylen in das zolithische Porengefüge eindringen kann und dort adsorbiert wird, während Propan von der Diffusion ausgeschlossen ist, das Molekularsieb passiert und in hoher Reinheit anfällt. Das Propylen wird thermisch desorbiert und ebenfalls in hoher Reinheit erhalten. Das Verfahren ist insbesondere für die Trennung von Propylen-Propan-Gemischen mit geringem Propylen-Anteil geeignet . Ausbeutemindernde Begleiterscheinungen wie Oligomerisierung oder Koksabscheidung werden weitgehend ausgeschaltet.

Die bekannten Prozesse sind nur beschränkt anwendbar oder sie erfordern aufwendige Maßnahmen, die nicht immer wirtschaftlich vertretbar sind. Es bestand daher die Aufgabe ein Verfahren zu entwickeln, das technisch einfach ist und insbesondere bei Produktionen im technischen Maßstab die Trennung von Propylen-Propan-Gemischen mit vertretbaren Mitteln erlaubt. Das zu entwickelnde Verfahren muß sich unabhängig von der Zusammensetzung der Propylen-Propan-Gemische und auch in Gegenwart reaktionsbedingter Begleitstoffe durchführen lassen. Es soll überdies wirtschaftlich sein und sich gegebenenfalls problemlos in die Abfolge mehrerer Reaktionsstufen einfügen.

Die Erfindung löst diese Aufgabe durch ein Verfahren zur Trennung von Propylen-Propan-Gemischen durch selektive Absorption. Es ist dadurch gekennzeichnet, daß als Absorptionsmittel Wasser verwendet wird.

Überraschenderweise gelingt es nach dem neuen Verfahren, Propylen-Propan-Gemische auf einfachem Wege und unter Verwendung technisch gebräuchlicher Vorrichtungen in ihre Komponenten aufzutrennen. Das Absorptionsmittel Wasser ist umweltverträglich und kostengünstig und steht in der Regel auch in erforderlicher Qualität zur Verfügung. Es wird auf Grund der Löslichkeitsverhältnisse nur in sehr geringen Mengen zusammen mit dem Propan ausgetragen. Die Kohlenwasserstoffe werden in einer Reinheit gewonnen, die den Anforderungen für die meisten Anwendungsgebiete genügt. Verluste durch chemische Umwandlungen der Kohlenwasserstoffe treten nicht auf.

Erfindungsgemäß erfolgt die Trennung von Propylen-Propan-Gemischen nach dem neuen Verfahren durch Extraktion mit Hilfe vor Wasser in der industriell üblicherweise zur Verfügung stehenden Qualität. Es kann Trinkwasser, Kondensat oder entionisiertes oder enthärtetes Wasser, wie auch Prozeßwasser vor- oder nachgeschalteter Verfahrensstufen verwendet werden. Vorzugsweise verwendet man entionisiertes Wasser.

Nach einer bevorzugten Ausführungsform des erfindungsgemäßen Verfahrens setzt man der Wasserphase Phasentransferreagenzien (auch Lösungsvermittler oder Tenside genannt) zu. Sie verändern das Lösungsvermögen für die beiden Kohlenwasserstoff-Komponenten und erhöhen dadurch den Trenneffekt.

Als Lösungsvermittler sind Verbindungen bekannt, deren hydrophile Gruppen ionisch (anionisch oder kationisch) oder nicht ionisch sind. Zu den anionenaktiven Verbindungen gehören Natrium-, Kalium- oder Ammoniumsalze von Carbonsäuren mit 8 bis 20 Kohlenstoffatomen, insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nicht ionischen Phasentransferreagenzien können in wäßriger Lösung nicht in Ionen dissoziieren. Zu ihnen zählen Alkylpolyethylenglykole, Alkylphenylpolyethylenglykole, Fettsäurealkylolamide und Trialkylaminooxide. Schließlich finden auch Ampholyte wie Aminocarbonsäuren, Betaine und Sulfobetaine als Lösungsvermittler Verwendung. Im Rahmen der Erfindung besonders bewährt haben sich ionische Tenside, unter ihnen als kationische Tenside Trimethyl-tetradecylammoniumsalze und anionische Tenside, unter ihnen Dodecylsulfonat.

Im allgemeinen können die zu trennenden Propylen-Propan-Gemische in der Form angewandt werden, in der sie bei den in Raffinerien oder in der chemischen Industrie ausgeübten Prozessen anfallen. In Ausnahmefällen kann es erforderlich sein, den Absorptionsprozeß störende oder belastende Bestandteile wie nicht oder nur unvollständig abgetrennte Produkte und Nebenprodukte vorausgehender Verfahrensstufen vor der Trennung des Kohlenwasserstoffgemischs zu entfernen.

Zur Durchführung der Absorption wird das Gasgemisch feinverteilt in das Absorptionsmittel eingeleitet und im Gegenstrom oder auch im Gleichstrom zum auf große Oberfläche ausgebreiteten oder versprühten Absorptionsmittel geführt. Hierbei tritt bevorzugt Propylen durch die Phasengrenze in die Flüssigkeit ein und bildet eine Mischphase, während sich das Trägergas, das sind die übrigen Komponenten des Gasgemisches, indifferent verhalten und infolge ihrer - verglichen mit Propylen - wesentlich geringeren Löslichkeit nahezu vollständig aus dem Absorptionsmittel austreten. Die Absorption wird einstufig oder mehrstufig, diskontinuierlich oder kontinuierlich in den bekannten Absorptionsvorrichtungen durchgeführt. Als Absorber geeignet sind Riesel- und Sprühtürme, Bodenkolonnen, Füllkörperkolonnen, Dünnschichtabsorber, Rotationswäscher und Rotationskolonnen. Die Absorption erfolgt innerhalb weiter Temperatur- und Druckbereiche. Bevorzugt werden Temperaturen zwischen -5 und 90°C, vorzugsweise 10 bis 50°C. Man kann drucklos arbeiten, erhöhte Drücke bis etwa 5,0 MPa, insbesondere von 4,0 bis 5,0 MPa erhöhen die Absorptionsleistung. Entsprechend seiner Löslichkeit wird insbesondere Propylen von der Wasserphase absorbiert, während Propan zusammen mit gegebenenfalls weiteren inerten gasförmigen Komponenten ungelöst aus dem Absorptionsmittel austreten.

Die Zahl der verwendeten Absorptionsstufen und damit die Vollständigkeit der Propylenabsorption ist abhängig von der Konzentration des Propylens im Propylen-Propan-Gemisch. Die Absorption erfolgt nach dem Henry'schen Gesetz und ist abhängig von der Konzentration der Kohlenwasserstoffe im Gemisch. Daher ist der Trennvorgang dann abzubrechen, wenn auf Grund zu geringer Propylen- und hoher Propankonzentration vom Wasser mehr Propan als Propylen aufgenommen wird.

Das im Absorptionsmittel gelöste Propylen wird in bekannter Weise z.B. durch Erhitzen, durch Druckminderung oder durch Austreiben mit Inertgas desorbiert. Die genannten Maßnahmen können einzeln, gemeinsam oder als Kombination von zwei Grundoperationen angewandt werden.

Nach einer besonderen Ausführungsform des erfindungsgemäßen Verfahrens wird das Propylen-Propan-Gemisch in einer kontinuierlich arbeitenden Absorptionskolonne im Gegenstrom zum Wasser geführt. Anschließend erwärmt man die Lösung von Propylen in Wasser, entspannt sie in einer Desorptionsvorrichtung, zieht den entweichenden ungesättigten Kohlenwasserstoff ab und leitet ihn der weiteren Verwendung zu. Das im Sumpf der Desorptionsvorrichtung anfallende propylenarme Wasser wird ebenfalls abgezogen, gekühlt, gegebenenfalls unter Nutzung des Wärmeinhalts, und in die Absorptionskolonne zurückgeführt. Entsprechend der Abhängigkeit der absorbierten Propylenmenge von der Propylenkonzentration im Gasgemisch läßt sich durch entsprechendes Bemessen der Wassermenge die Propankonzentration im abgetrennten Propylen und die Propylenkonzentration im abgetrennten Propan auf gewünschte Werte einstellen.

Das neue Verfahren ist in allen Fällen anwendbar, in denen Propylen und Propan voneinander zu trennen sind. Diese Aufgabe stellt sich z.B. bei der Hydroformylierung von Propylen, bei der u.a. ein Propylen-Propan-Gemisch als Abgas anfällt. Insbesondere bei der Verwendung in Wasser gelöster, heterogener Katalysatoren, z.B. Rhodium-Phosphin-Komplexverbindungen, hat sich die beanspruchte Arbeitsweise bewährt, weil das Absorptionsmittel Wasser auch Reaktionsmedium bei der Umsetzung von Olefin mit Synthesegas ist.

Durch das nachfolgende Beispiel wird die beanspruchte Arbeitsweise näher beschrieben, jedoch nicht auf die angegebene Ausführungsform beschränkt.

### Beispiel

Dem unteren Teil einer Gegenstrom-Absorptionskolonne mit 10 theoretischen Stufen werden je Stunde 1853 kg eines Gasgemisches zugeführt, das neben anderen Bestandteilen 60 Masse-% Propylen und 22 Masse-% Propan enthält und dessen Temperatur 51°C beträgt. Vom Kopf der Absorptionskolonne, die bei 4,0 MPa betrieben wird, strömen dem Gasgemisch je Stunde 130 m³ Wasser von 10°C als Absorptionsmittel entgegen. Am Kopf der Absorptionskolonne werden je Stunde 586 kg eines Gasgemisches entnommen, das 53 Masse-% Propan und 1,3 Masse-% Propylen enthält. Am Sumpf der Kolonne wird das mit Propylen beladene Absorptionsmittel abgezogen, auf 70°C erwärmt, dem Kopf einer bei 1,0 MPa betriebenen Desorptionskolonne mit 3 theoretischen Stufen zugeführt und entspannt. Am Sumpf der Desorptionskolonne werden je Stunde 30 kg Synthesegas von 15°C eingespeist und am Kopf je Stunde 1312 kg eines Gasgemisches entnommen, das 83 Masse-% Propylen und 8 Masse-% Propan enthält. Das von Propylen befreite Wasser wird gekühlt und wieder der Absorptionskolonne zugeführt. Das propylenreiche Gas kann unmittelbar genutzt werden.

## Patentansprüche

1. Verfahren zur Trennung von Propylen-Propan-Gemischen durch selektive Absorption, dadurch gekennzeichnet, daß als Absorptionsmittel Wasser verwendet wird.

2. Verfahren nach Anspruch 1, dadurch gekennzeichnet, daß als Absorptionsmittel entionisiertes Wasser verwendet wird.

3. Verfahren nach Anspruch 1 oder 2, dadurch gekennzeichnet, daß dem Wasser ein Phasentransfermittel zugesetzt wird.

4. Verfahren nach Anspruch 3, dadurch gekennzeichnet, daß das Phasentransfermittel Trimethyltetradecylammoniumsalz oder Dodecylsulfonat ist.

5. Verfahren nach einem oder mehreren der Ansprüche 1 bis 4, dadurch gekennzeichnet, daß die Absorption bei Temperaturen zwischen -5 und 90°C, vorzugsweise 10 bis 50°C erfolgt.

6. Verfahren nach einem oder mehreren der Ansprüche 1 bis 5, dadurch gekennzeichnet, daß die Absorption bei Drücken bis etwa 5,0 MPa, insbesondere 4,0 bis 5,0 MPa erfolgt.
